# EUROPÄISCHE PATENTSCHRIFT

(11) **EP 1 326 530 B1**
(45) Veröffentlichungstag und Bekanntmachung des Hinweises auf die Patenterteilung: **25.06.2008**
(21) Anmeldenummer: 01987648.1
(22) Anmeldetag: 22.10.2001
(51) Int. Cl.: A61B 5/00, A61B 5/103

(54) **Verfahren und Vorrichtung zur Identifizierung eines zu behandelnden Auges bei Operationen**
Method and device for identifying an eye that is to be treated in operations
Procédé et dispositif pour identifier un oeil devant être traité lors d'opérations

(30) Priorität: 20.10.2000 DE 10052201
(43) Veröffentlichungstag der Anmeldung: 16.07.2003
(73) Patentinhaber: Carl Zeiss Meditec AG, 07745 Jena (DE)
(72) Erfinder: FEIGE, Torsten, 07743 Jena (DE); MÄUSEZAHL, Holger, 07745 Jena (DE)
(74) Vertreter: Schnekenbühl, Robert Matthias L.
(86) Internationale Anmeldenummer: PCT/EP2001/012171
(87) Internationale Veröffentlichungsnummer: WO 2002/032351

(56) Entgegenhaltungen:
- WO-A-99/00690
- US-A- 5 749 361
- US-A- 6 069 689

## Beschreibung

Die vorliegende Erfindung betrifft ein Verfahren und eine Vorrichtung zur Identifizierung eines zu behandelnden Operationsgebietes, insbesondere eines Auges bei Laseroperationen.

Bei Laseroperationen, insbesondere eines Auges, werden Daten für die Laseransteuerung benötigt, die die individuelle Behandlung eines Auges zulassen. Diese Daten werden üblicherweise als Patientendaten erfasst bzw. ermittelt. Hierzu dienen Methoden wie Sehtest, Refraktometer, Erfassung der Topografie, beispielsweise der Hornhaut oder der Linse sowie die Aberration des Auges. Diese Daten werden abgespeichert, sei es rechnergestützt oder per Hand in einer Akte. Solche Daten umfassen Name, Sphere, Zylinder, Achse, Auge (links/rechts), Topografie, Aberration, etc.

In einem späteren Schritt werden diese Daten eingegeben (Sphere, Zylinder, Achse) bzw. die Schusskoordinaten für den Operationslaser geladen, die aus Berechnungen von topografiegestützten Daten bzw. Ableitung von Koordinaten aus Messung der Abberationen des Auges gewonnen wurden. Danach wird die Laseroperation auf der Grundlage der eingegebenen Daten durchgeführt.

Es besteht somit eine räumliche und zeitliche Trennung zwischen Ermittlung der Daten und Anwendung dieser Datensätze. Die Zuordnung zwischen den Daten und dem Patient bzw. dem einzelnen Auge erfolgt über die persönlichen Daten des Patienten wie Name, Vorname, Geburtsdatum, etc.

Aufgrund der bestehenden räumlichen und zeitlichen Trennung zwischen Erfassung der Daten und Nutzung dieser für eine Laseroperation ist ein Zusammenhang zwischen dem Patientenauge, an dem die Daten gewonnen wurden, und dem Patientenauge, an dem die Operation durchgeführt werden soll, nur über Hilfsmittel gegeben. Diese sind vorwiegend Name des Patienten, ID-Nummer der Arztpraxen und Angaben von welchem Auge (links oder rechts) die Daten gewonnen wurden.

Es ist daher möglich, dass es zu einer falschen Zuordnung zwischen dem Datensatz der Korrektur und dem konkret operierten Auge kommt. Dies kann Ursachen in der eingesetzten Systemsoftware zur Verwaltung der Datensätze haben oder auch menschliches Versagen sein. Insbesondere kann es selbst bei sorgfältigster Verfahrensweise bei der Datenerhebung leicht zur Verwechslung von rechten und linken Augen beim selben Patienten kommen. Auch ist eine Verwechslung von Patienten untereinander (Namensähnlichkeit, Unaufmerksamkeit bei der Datenerfassung, etc.) möglich.

Aufgabe der vorliegenden Erfindung war es daher, ein Verfahren und eine Vorrichtung zur Identifizierung eines Operationsgebietes insbesondere eines Auges, bei einer Laseroperation bereitzustellen, das die Gefahr von Operationen am falschen Operationsgebiet bzw. Auge verhindern.

Ein Beispiel des Standes der Techik ist in US-A-6 069 689 beschrieben.

Die Aufgabe der vorliegenden Erfindung wurde durch das Verfahren bzw. die Vorrichtung nach den unabhängigen Ansprüchen gelöst. Vorteilhafte Weiterbildungen sind in den abhängigen Ansprüchen definiert.

Insbesondere wird die Aufgabe durch ein Verfahren zur Identifizierung eines Operationsgebietes bei einer Operation, insbesondere unter Verwendung eines Lasersystems, anhand eindeutiger Daten über das Operationsgebiet gelöst, wobei eine erste Kopie der eindeutigen Daten im Lasersystem abgelegt wird, die eindeutigen Daten in unmittelbarer Umgebung des Operationsgebietes und/oder am Operationsgebiet angeordnet sind, vor Beginn der Operation die erste Kopie an die eindeutigen Daten auf ihre Identität überprüft werden und das Ergebnis der Identitätsprüfung an das Lasersystem weitergegeben wird. Dadurch ist es möglich, dass das einzelne Operationsgebiet, vorzugsweise das Auge, eindeutig vor der Operation als das korrekte identifiziert wird, für das die Schusspositionen im Laser vorgesehen sind. Auf diese Weise wird vermieden, dass ein Patient mit den falschen Schussdaten behandelt wird und somit Korrekturen an beispielsweise seinem Auge vorgenommen werden, die für einen anderen Patienten berechnet worden sind.

Als Lasersystem werden Operationssysteme mit einem Laser, beispielsweise einem Excimerlaser wie z.B. einem Spotscanning-Excimerlaser verstanden. Diese Lasersysteme umfassen bevorzugt auch Rechner, um die Behandlung rechnergestützt durchzuführen. Besonders bevorzugt sind ebenfalls Zusatzsysteme wie Eye-Tracking-Systeme vorgesehen sowie Beleuchtungseinrichtungen des Operationsgebietes, d.h. insbesondere des Auges.

Die eindeutigen Daten sind eindeutig in Bezug auf das Operationsgebiet. Ein Operationsgebiet kann von einem anderen Operationsgebiet daher eindeutig aufgrund dieser eindeutigen Daten unterschieden werden.

Die erste Kopie der eindeutigen Daten ist eine Kopie des relevanten, das Auge eindeutig identifizierenden Datengehaltes. Diese erste Kopie kann im Lasersystem abgelegt werden und steht so dem Lasersystem im späteren Operationsablauf zur Verfügung. Besonders bevorzugt werden diese eindeutigen Daten vor der Operation ermittelt und stehen dann im weiteren Operationsverlauf zur Verfügung.

Die eindeutigen Daten sind besonders bevorzugt in unmittelbarer Umgebung des Operationsgebietes und/oder am Operationsgebiet angeordnet. Besonders bevorzugt sind diese eindeutigen Daten daher unmittelbar neben dem Auge oder sogar im Auge vorhanden. Es kann sich hier bevorzugt um extra aufgebrachte Daten handeln oder aber um Daten, die im Operationsgebiet bzw. Auge schon direkt vorliegen. So ist es beispielsweise möglich, diese eindeutigen Daten räumlich und zeitlich fest an das Auge zu binden, beispielsweise über einen Aufkleber, eine Farbmarkierung, bevorzugt als Strichcode, ein fest mit der Augenbraue verbundener Mikrochip, etc. oder es handelt sich um eindeutige Daten, die bereits am Operationsgebiet angeordnet sind, d .h. um fixe Parameter, die dieses Operationsgebiet, insbesondere das Auge, eindeutig definieren, wie ein Fingerabdruck einen Daumen definiert.

Bevorzugt wird dann vor Beginn der Operation die erste Kopie der eindeutigen Daten mit den eindeutigen Daten verglichen und so eine Identitätsprüfung vorgenommen. Das Ergebnis dieser Identitätsprüfung wird dann, bevorzugt an das Lasersystem weitergegeben. Von dem Ergebnis dieser Identitätsprüfung kann dann das weitere Vorgehen abhängig gemacht werden. So ist es beispielsweise möglich, die Operation erst gar nicht zuzulassen, wenn die Identitätsprüfung nicht erfolgreich stattgefunden hat. Es ist außerdem denkbar, den Operateur vor Beginn der Operation auf ein Fehlschlagen der Identitätsprüfung hinzuweisen.

Nach einem bevorzugten Ausführungsbeispiel der vorliegenden Erfindung umfassen die eindeutigen Daten Namen des Patienten, Vorname des Patienten, Geburtsdaten des Patienten, Patienten-ID, Arzt-ID, Angaben über das Operationsgebiet oder eine Kombination einzelner oder aller dieser Daten. So ist es beispielsweise möglich, eine Zeichenfolge zu generieren, die Informationen über das spezielle Auge eines speziellen Patienten umfassen und dieses so eindeutig zu machen. Besonders bevorzugt wird jedoch eine Angabe über das Operationsgebiet selbst verwendet, insbesondere Gefäßzeichnungen und/oder Zeichnungen der Iris. Besonders bevorzugt werden dazu Gefäßzeichnungen des Augenhintergrundes genutzt, die bei jedem Auge, selbst desselben Patienten, unterschiedlich ist und höchst individuelle Charakteristika aufweist, die das Auge eindeutig identifizieren. Weiterhin besonders bevorzugt ist die Zeichnung der Iris. Diese Zeichnungen sind eindeutig sowie zeitlich und räumlich stabil. Ein weiterer Vorzug dieser Angaben über das Auge ist, dass viele Lasersysteme bereits Erfassungsgeräte mit Bildaufnahmemöglichkeiten und bildverarbeitenden Komponenten beinhaltet (Topografiegeräte, Aberrometer, etc.). So können besonders bevorzugt zeitgleich mit der Datenerfassung das Merkmal bzw. die eindeutigen Daten erfasst und mit dem Datensatz gespeichert werden. Auf der Seite des Operationsgerätes sind häufig auch bildverarbeitende Systeme (Eye-Tracking-Systeme) eingesetzt. Mittels dieser Systeme kann das Merkmal bzw. die eindeutigen Daten nun aus dem der Operation aktuell vorliegenden Auge ermittelt und mit dem Merkmal im Datensatz verglichen werden. Ist das Merkmal identisch so ist die eindeutige Zuordnung gegeben.

Bei besonders bevorzugten Ausführungsbeispielen der vorliegenden Erfindung sind die eindeutigen Daten als Strichcode, Microchip, Zeichenfolgen oder einer Kombination dieser Darstellungsweisen verkörpert. Diese eindeutigen Daten, die dann bevorzugt in unmittelbarer Umgebung des Operationsgebietes angeordnet werden, können beispielsweise aufgeklebt, aufgezeichnet, aufgetragen bzw. aufgemalt werden.

Weiterhin wird die Erfindung durch eine Vorrichtung zur Identifizierung eines Operationsgebietes bei einer Operation, besonders bevorzugt zur Ausführung eines der erfindungsgemäßen Verfahren gelöst, umfassend ein Lasersystem mit einer Speichereinheit mit einer Speicherung eindeutiger Daten über das Operationsgebiet und einer Lasereinheit, wobei die eindeutigen Daten mit der Lasereinheit erfassbar sind, eine erste Kopie der eindeutigen Daten in der Speichereinheit ablegbar ist, und eine Vergleichseinrichtung vorgesehen ist, mit der die erste Kopie die eindeutigen Daten auf ihre Identität überprüfbar sind. Mit einer solchen Vorrichtung ist sowohl die Erfassung eindeutiger Daten über eine Lasereinheit, wie beispielsweise einem Strichcodelaser oder einer Bilderfassungseinheit, möglich und es können diese eindeutigen Daten in Kopie in der Speichereinheit abgelegt werden, so dass sie über eine Vergleichseinrichtung später mit den eindeutigen Daten überprüft werden können.

Mit dieser Vorrichtung ist gewährleistet, dass die gewonnenen Daten immer eindeutig zu dem Auge, von dem diese gewonnen wurden, zugeordnet werden.

Namensverwechslungen, etc. spielen keine Rolle mehr, da primär immer die Daten an das Auge gebunden sind und nicht an den Namen. Diese Lösungen können auch bevorzugt aktiv oder passiv genutzt werden.

Bei einer passiven Nutzung wird der Datensatz wie bisher über Patientennamen etc. ausgewählt, unmittelbar danach wird durch die Lasereinheit bzw. eine Identifizierungseinheit das Merkmal bzw. die eindeutigen Daten am Patientenauge gesucht und mit dem Datensatz verglichen. Im Fall einer Unstimmigkeit zwischen den Merkmalen wird eine Operation nicht zugelassen.

Bei einer aktiven Nutzung wird das Patientenauge direkt durch die Lasereinheit bzw. die Identifizierungseinheit untersucht und der dazu passende Datensatz automatisch in den Laser geladen. Dem Arzt werden diese Patientendaten (wie Name etc.) bevorzugt dann auch angezeigt.

Weiterhin ist es möglich, die Erfindung mit einer Aufbringung der eindeutigen Daten bzw. dieses zusätzlichen Merkmals zu verwenden oder aber diese eindeutigen Daten direkt aus dem Auge selbst zu gewinnen.

Besonders bevorzugt wird der Augenkorrekturzustand zusätzlich nochmals kurz vor der Operation überprüft. Auf diese Weise ist es zusätzlich möglich, Doppeloperationen am selben Auge zu vermeiden.

Im Folgenden werden anhand von Zeichnungen vorteilhafte Weiterbildungen der vorliegenden Erfindung beschrieben. Es zeigen:
- Figur 1:: eine schematische Darstellung eines Ausführungsbeispiels des erfindungsgemäßen Verfahrens;
- Figur 2:: ein Ausführungsbeispiel zur Anbringung der eindeutigen Daten in Augennähe und
- Figur 3:: eine schematische Darstellung eines Ausführungsbeispiels der vorliegenden Erfindung als Datenerfassungs- bzw. Behandlungsgerät.

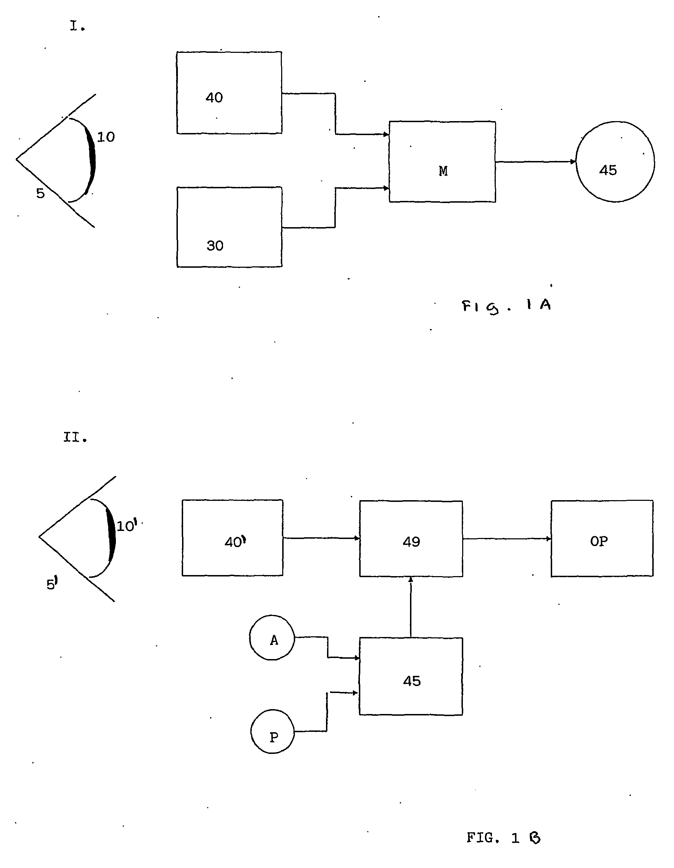

In Figur 1 ist eine schematische Darstellung eines Ausführungsbeispiels des erfindungsgemäßen Verfahrens dargestellt, bei der in einem ersten Schritt I ein Auge 5 mit einem entsprechenden eindeutigen Merkmal 10 durch eine Lese- bzw. Identifikationseinheit 40 abgetastet wird und die eindeutigen Daten 10 bzw. das Merkmal - hier beispielsweise die Struktur der Iris - erfaßt wird. Über eine Schußdatenerfassungseinheit 30 werden die vorhandenen Fehler des Auges 5 bzw. die beabsichtigten Grundlagen für die Schußdaten zur Korrektur des Auges als Datensatz erfaßt. Diese beiden Ergebnisse werden in einer Mischstufe M zusammengefügt, so daß die eindeutigen Daten bzw. das Merkmal zum Schußdatensatz hinzugefügt werden. Hinzu können noch die Erfassung von Patientendaten treten, die über eine weitere Eingabeeinheit (nicht dargestellt) ebenfalls zu dem Datensatz hinzugefügt werden können. Dieser gesamte Datensatz wird dann in der Speichereinrichtung 45 abgelegt.

In einem zweiten Schritt II wird später das konkret für die Operation vorliegende Auge 5' mit dem eindeutigen Merkmal 10' der Irisstruktur abgetastet und das eindeutige Merkmal 10' in der Identifikationseinheit 40' erfaßt. In der Speichereinrichtung 45 werden dann entweder über die vorher gespeicherten Schußdaten die zu dem Merkmal passenden Schußdaten in die Speichereinrichtung 45 geladen und/ oder über eine direkte Eingabe der Daten durch den Arzt wie Sphere, Abberation, etc. diese an die Speichereinrichtung 45 übergeben. In der Vergleichseinrichtung 49 werden dann die vom konkret vor dem Laser liegenden Auge 5' ermittelte eindeutige Merkmal 10' und das in der Speichereinrichtung 45 vorhandene vorher in Schritt I zu dem vorliegenden Schußdatensatz ermittelte Merkmal 10 verglichen. Wenn die beiden Merkmal 10 und 10' übereinstimmen, wird von der Vergleichseinrichtung 49 ein Signal ausgegeben, das die Operation OP freigibt.

Auf diese Weise wird anhand von im oder am Auge 5' vorliegende eindeutige Daten bzw. Merkmal 10' die Zusammengehörigkeit zu dem für die Operation vorliegenden Schußdaten verifiziert und hierdurch eine besondere Sicherheit vor Verwechslungen oder Fehlzuweisungen bei der folgenden Operation vermieden.

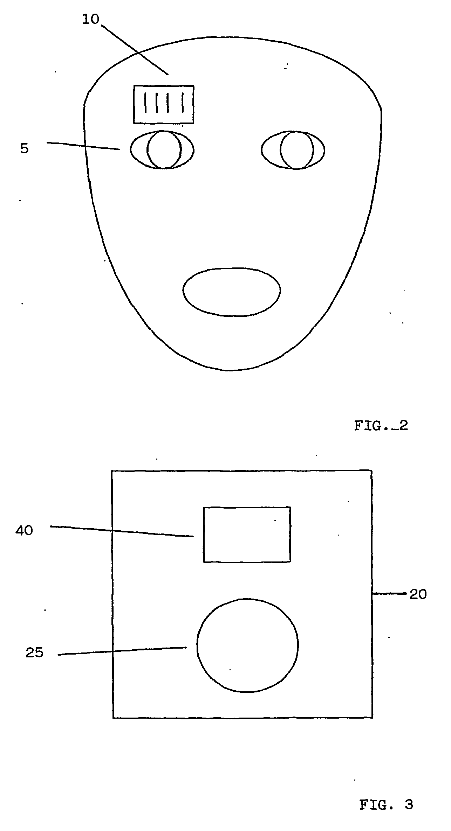

In Figur 2 ist ein Ausführungsbeispiel zur Anbringung der eindeutigen Daten in Augennähe dargestellt. Das eindeutige Merkmal 10 ist hierbei als Strichcode über dem Auge 5 in Höhe der Augenbraue fest angebracht, vorzugsweise aufgeklebt. Auf diese Weise kann das im Strichcode verkörperte eindeutige Merkmal bzw. die eindeutigen Daten 10 sowohl bei der Erfassung der Korrekturdaten in Schritt I sowie auch bei der Überprüfung in Schritt II erfaßt werden.

In Figur 3 ist eine schematische Darstellung eines Ausführungsbeispiels der vorliegenden Erfindung als Datenerfassungs- bzw. Behandlungsgerät dargestellt. Hierbei ist neben der Operationsstrahleinheit 25, aus der der Abtaststrahl zum Ermitteln der Korrekturdaten in Schritt I bzw. der Laserstrahl, mit dem die Operation in Schritt II durchgeführt wird, eine Leseeinheit bzw. Identifikationseinheit 40 vorgesehen, über die das eindeutige Merkmal 10 erfaßt werden kann. Besonders bevorzugt wird diese Leseeinheit 40 durch Geräte im Lasersystem 20 dargestellt, die sowieso schon bei herkömmlichen Lasersystemen vorhanden sind wie beispielsweise Topografiegeräte und Abberometer in Schritt I bzw. Eyetracker in Schritt II.

## Patentansprüche

1. Verfahren zur Identifizierung eines Operationsgebietes (1) bei einer Operation, insbesondere unter Verwendung eines Lasersystems (20), anhand eindeutiger Daten (10) über das Operationsgebiet (1), wobei
eine erste Kopie (11) der eindeutigen Daten (10) im Lasersystem (20) abgelegt wird,
**dadurch gekennzeichnet, dass**
die eindeutigen Daten (10) in unmittelbarer Umgebung des Operationsgebietes (1) und/oder am Operationsgebiet (1) angeordnet sind,
vor Beginn der Operation die erste Kopie (11) und die eindeutigen Daten (10) auf ihre Identität überprüft werden und
das Ergebnis der Identitätsprüfung an das Lasersystem (20) weitergegeben wird.

2. Verfahren nach dem vorhergehenden Anspruch,
**dadurch gekennzeichnet, dass**
das Operationsgebiet (1) ein Auge (5) ist.

3. Verfahren nach einem der vorhergehenden Ansprüche,
**dadurch gekennzeichnet, dass**
die eindeutigen Daten (10) Name des Patienten, Vorname des Patienten, Geburtsdatum des Patienten, Patienten ID, Arzt ID, Angaben über das Operationsgebiet (1) oder eine Kombination einzelner oder aller dieser Daten umfassen.

4. Verfahren nach dem vorhergehenden Anspruch,
**dadurch gekennzeichnet, dass**
die Angaben über das Operationsgebiet (1) Gefäßzeichnungen und/ oder Zeichnungen der Iris umfassen.

5. Verfahren nach einem der vorhergehenden Ansprüche,
**dadurch gekennzeichnet, dass**
die eindeutigen Daten (10) als Strichcode, Microchip, Zeichenfolgen oder einer Kombination dieser Darstellungsweisen verkörpert sind.

6. Vorrichtung zur Identifizierung eines Operationsgebietes bei einer Operation, umfassend ein Lasersystem (20) mit einer Speichereinheit (45) zur Speicherung eindeutiger Daten (10) über das Operationsgebiet (1) und einer Leseeinheit (40)
**dadurch gekennzeichnet, dass**
die eindeutigen Daten (10) mit der Leseeinheit (40) erfasst werden,
eine erste Kopie (11) der eindeutigen Daten (10) in der Speichereinheit (45) ablegt ist und
eine Vergleichseinrichtung (49) vorgesehen ist, mit der die erste Kopie (11) und die eindeutigen Daten (10) auf ihre Identität überprüfbar sind.

7. Verwendung eines Lasersystems (20) zur Ausführung eines Verfahrens nach einem der vorhergehenden auf ein Verfahren gerichteten Ansprüche.

## Claims

1. Method of identifying an operative field (1) during an operation, in particular using a laser system (20), with the help of unambiguous data (10) relating to the operative field (1), wherein
a first copy (11) of the unambiguous data (10) is stored in the laser system (20), **characterized in that**
the unambiguous data (10) are arranged in the immediate vicinity of the operative field (1) and/or on the operative field (1),
before the start of the operation the first copy (11) and the unambiguous data (10) are checked to ensure they are identical and
the result of the identity check is forwarded to the laser system (20).

2. Method according to the previous claim,
**characterized in that**
the operative field (1) is an eye (5).

3. Method according to one of the previous claims,
**characterized in that**
the unambiguous data (1) comprise the patient's name, the patient's first name, the.patient's date of birth, the patient's ID, the doctor's ID, details about the operative field (1) or a combination of individual items or all of these data.

4. Method according to the previous claim,
**characterized in that**
the details about the operative field (1) comprise drawings of vessels and/or drawings of the iris.

5. Method according to one of the previous claims,
**characterized in that**
the unambiguous data (10) are represented as a bar code, microchip, character strings or a combination of these representation methods.

6. Device for identifying an operative field during an operation, comprising a laser system (20) with a memory unit (45) for storing unambiguous data (10) about the operative field (1) and a reading unit (40)
**characterized in that**
the unambiguous data (10) are recorded by the reading unit (40),
a first copy (11) of the unambiguous data (10) is stored in the memory unit (45) and
a comparison device (49) is provided with which the first copy (11) and the unambiguous data (10) can be checked to ensure they are identical.

7. Use of a laser system (20) to carry out a method according to one of the previous claims directed towards a method.

## Revendications

1. Procédé pour identifier un champ opératoire (1) lors d'une opération,utilisant notamment un système laser (20), à l'aide de données univoques (10) sur le champ opératoire (1),
une première copie (11) des données univoques (10) étant mémorisée dans le système laser (20),
**caractérisé en ce que**
les données univoques (10) sont disposées dans l'environnement immédiat du champ opératoire (1) et/ou dans le champ opératoire (1) ;
une vérification de l'identité de la première copie (11) et des données univoques (10) est effectuée avant le début de l'opération ; et
le résultat du contrôle d'identité est transmis au système laser (20).

2. Procédé selon la revendication précédente,
**caractérisé en ce que** le champ opératoire (1) est un oeil (5).

3. Procédé selon l'une quelconque des revendications précédentes,
**caractérisé en ce que**
les données univoques (10) comprennent le nom du patient, le prénom du patient, la date de naissance du patient, l'identité du patient, l'identité du médecin, des indications sur le champ opératoire (1) ou une combinaison de tout ou partie de ces données.

4. Procédé selon la revendication précédente,
**caractérisé en ce que**
les indications sur le champ opératoire (1) comprennent des dessins du système vasculaire et/ou des dessins de l'iris.

5. Procédé selon l'une quelconque des revendications précédentes,
**caractérisé en ce que**
les données univoques (10) sont matérialisées sous la forme d'un code à barres, d'une micropuce, de séquences de caractères ou d'une combinaison de ces types de représentation.

6. Dispositif pour identifier un champ opératoire lors d'une opération, comprenant un système laser (20) avec une unité de mémorisation (45) pour mémoriser des données univoques (10) relatives au champ opératoire (1) et une unité de lecture (40),
**caractérisé en ce que**
les données univoques (10) sont saisies avec l'unité de lecture (40) ;
une première copie (11) des données univoques (10) est mémorisée dans l'unité de mémorisation (45) ; et
un dispositif comparateur (49) est prévu avec lequel on vérifie l'identité de la première copie (11) et des données univoques (10).

7. Utilisation d'un système laser (20) pour mettre en oeuvre un procédé selon l'une quelconque des revendications précédentes relatives à un procédé.
